# EUROPEAN PATENT APPLICATION

(11) **EP 2 567 669 A1**
(43) Date of publication of application: **13.03.2013**
(21) Application number: 12182936.0
(22) Date of filing: 04.09.2012
(51) Int. Cl.: A61B 19/00, A61B 10/06, A61B 17/00

(54) **Surgical tool for use in MR imaging**

(30) Priority: 09.09.2011 US 201113229264
(71) Applicant: Imris Inc., Winnipeg, Manitoba R3T 1N5 (CA)
(72) Inventor: Sutherland, Garnette, Calgary, Alberta T2N 4Z6 (CA); Klassen, James, Langley, Alberta V2Y 1N5 (CA)
(74) Representative: Jostarndt, Hans-Dieter

(57) **Abstract**

A biopsy tool for use in MR imaging for operation by a robot arm is formed of relatively brittle ceramic materials which have a magnetic susceptibility which is substantially equal to that of human tissue. The tool has slip couplings and bend joints designed to prevent overloading of forces on the sampling jaws. Cleaning ports are integrated into the design so that sterility can be obtained by flushing the device interior with a cleaning fluid. A novel spring-loaded capstan operated by a crank movable longitudinally of the tool ensures proper cable tension. A unique jaw shape enables a cutting pressure to be applied simultaneously around the desired tissue and does not depend on sharp edges to obtain the sample. Springs in the main casing provide cable tensioning to keep the jaws in a default closed position for movement of the biopsy device along a trajectory to the sample to be acquired.

## Description

This invention relates to a surgical tool for use in MR imaging.

### BACKGROUND OF THE INVENTION

Traditional surgery relies on the physician's surgical skills and dexterity and ability to localize structures in the body. Surgical robots have recently been developed to address the physical human issues such as fatigue and tremor in procedures. These systems were specifically developed for Minimally Invasive Surgery (MIS) or "key-hole" general surgery, orthopaedics and stereotactic neurosurgery.

Surgical robots have the potential to increase the consistency and quality of neurosurgery, and when used in conjunction with the advanced diagnostic imaging capabilities of MRI, can offer dramatic improvements. The Intuitive Surgical Inc. da Vinci and Computer Motion ZEUS robots are examples of MIS robots.

Unfortunately, there are no surgical robots that uses updated or real time MRI patient data to achieve accurate image-guided surgery. MR provides excellent soft tissue contrast for brain surgery and angiography. However MR can be expensive; can be slow to image; limits the tools that can be used which must be MR compatible; requires RF quiet environment; line of sight limits for in-bore use; and mounts a large object in the OR.

Given the superior tissue contrast provided by MR, there is clinical motivation to use MR images to guide surgical actions.

Note that: "The interventional MRI safety issues that exist for a surgical instrument include unwanted movement caused by magnetic field interactions (i.e., the missile effect), heating generated by radiofrequency (RF) power deposition, and artifacts associated with the use of the instrument, if it is in the imaging area of interest during its intended use." See Shellock - JOURNAL OF MAGNETIC RESONANCE IMAGING 13:152-157 (2001).

Uses of neurosurgical tools, such as those used in the present invention, include: dissecting/tissue manipulation, cutting, ablation/cauterizing, biopsy, aspiration. For example, biopsies acquire a small portion of tissue of interest from a specific region in a patient. The biopsy procedure is termed stereotactic due to the precise localization with which the sample is obtained. Multiple biopsies may be taken from a single patient.

In US patent 7,156,316 (Sutherland et al) issued December 26 2006 discloses a surgical tool for use in an MR imaging system but discloses that the tool should be manufactured of titanium which is well known for use in the high magnetic field and high RF field associated with MR. This tool is particularly designed for use in relation to a Robotic surgical device. The disclosure of this patent is hereby incorporated herein by reference.

The present invention relates to a tool for use in an MR imaging system and may be used in a robotic surgical system of the type disclosed in the above patent or may be used in a manual system where the tool is manipulated by the surgeon, often using guidance systems.

In US patent 7,391,173 (Schena) issued June 24 2008 and assigned to Intuitive Surgical is disclosed an actuation system for a surgical tool which includes first and second motor driven capstans for operating a cable drive to the tool head.

In US published application 2008/0009838 (Schena) published January 10 2008 and assigned to Intuitive Surgical is disclosed an actuation system for a surgical tool which includes a compact capstan system.

In US published application 2010/0082041 (Prisco) published April 1 2010 and assigned to Intuitive Surgical is disclosed an actuation system for a surgical tool which includes a motor driven capstan system with a tendon driven by the capstan and having an end attached to a passive pre-load system.

### SUMMARY OF THE INVENTION

It is one object of the invention to provide a surgical tool for use in MR imaging.

According to one aspect of the invention there is provided a surgical tool for use on a patient in an MR Imaging system comprising:
a tool support member;
the tool support member having a first end carrying an operating device for carrying out a procedure on a part of the patient;
the tool support member having a second end including an actuation device for actuating the operating device;
the tool being formed of a material which:
   has a minimal impact on MR images due to the lack of MR spin signal in the material;
   is non-ferromagnetic so as to be unresponsive to a magnetic field of the MR imaging system;
   is non-conductive of electric current so as to be unresponsive to an RF field of the MR imaging system so as to avoid heating of the tool by the RF field;
   has selected components having a magnetic susceptibility which is substantially equal to that of human tissue.

In one arrangement, the actuation device is arranged to be actuated by an end effector of a robot. However the actuation device can also be arranged to be actuated manually.

Preferably there is provided a force limiting component arranged to limit force applied to the operating device by the actuation device to a predetermined maximum force.

Preferably the force limiting component includes a drive transfer member which allows slippage of drive from the actuation device to the operating device.

Preferably the drive transfer member comprises an elongate band movable along its length by the actuation device, wherein the band is arranged to slip on a drive coupling around which it is wrapped.

Preferably the drive coupling is connected to the operating device for movement thereof between different positions thereof for carrying out the procedure on the part of the patient.

Preferably the elongate member is driven along its length by rotary capstan member at the actuation device around which the band is wrapped where the capstan member is rotated by a crank driven by movement of an engagement device longitudinally of the tool support member.

Preferably the crank engages one of inner and outer coaxial members with the capstan member housed within a housing connected to the other of the inner and outer coaxial members.

Preferably the capstan member is biased along the tool head relative to the housing by a pair of springs engaged between the housing and an axle of the capstan member.

Preferably the capstan member is driven by an actuation method that does not depend on electricity.

Preferably the tool support member is carried on a tool holder and wherein there is provided a force limiting component arranged to limit bending force applied to the tool support member by the tool holder to a predetermined maximum force.

Preferably the joint in the tool support member is pulled into engagement of the parts in the straight position by a band extending along the tool support member which extends against a spring tension to allow longitudinal movement of the parts into the bent position.

Preferably movement of the band operates the operating device.

Preferably the band extends along a hollow interior of the tool support member.

Preferably the tool support member and the operating device are formed at least in part of a ceramic material.

Preferably the ceramic material comprises a material selected from the group consisting of Yttrium zirconia, types of alumina, silicon nitride, and alloys thereof and which have a magnetic susceptibility substantially equal to that of human tissue.

Preferably the magnetic susceptibility of the material of the tool support member adjacent to and touching human tissue is substantially equal to that of human tissue

Preferably the operating device is driven by the actuation device through an actuation method that does not depend on electricity that could pose a safety hazard to the patient or that might introduce RF noise.

Preferably the biopsy tool includes a biopsy sample acquisition method that does not rely on sharp edges of a jaw.

Preferably the biopsy tool the operating device comprises a biopsy tool which includes cooperating jaws having a fixed jaw and a movable jaw, one of the jaws having a raised contact area fully surrounding a cup for receiving a biopsy sample with the contact area arranged to engage a cooperating surface of the other of the jaws, the jaws being arranged to provide an even application of closing force around the contact area between the jaws.

Preferably said raised contact area arranged to engage a planar cooperating surface of the other of the jaws so that the raised contact area forms a cutting edge on the planar surface.

Preferably the planar surface includes a cup facing the cup of said one jaw.

The arrangement described herein is primarily for use as a biopsy tool for use in a MRI scanner. However the features described herein can be applied to other surgical robotic instruments.

According to a second aspect of the invention there is provided a surgical tool for use on a patient in an MR Imaging system comprising:
a tool support member;
the tool support member having a first end carrying an operating device for carrying out a procedure on a part of the patient;
the tool support member having a second end including an actuation device for actuating the operating device;
the tool being formed of a material which:
   has a minimal impact on MR images due to the lack of MR spin signal in the material;
   is non-ferromagnetic so as to be unresponsive to a magnetic field of the MR imaging system;
   is non-conductive of electric current so as to be unresponsive to an RF field of the MR imaging system so as to avoid heating of the tool by the RF field;
the operating device being driven by an elongate band movable along its length by the actuation device;
wherein the band is arranged to slip on a drive coupling around which it is wrapped to provide a force limiting component arranged to limit force applied to the operating device by the actuation device to a predetermined maximum force;
wherein the drive coupling is connected to the operating device for movement thereof between different positions thereof for carrying out the procedure on the part of the patient.

According to a third aspect of the invention there is provided surgical tool for use on a patient in an MR Imaging system comprising:
a tool support member;
the tool support member having a first end carrying an operating device for carrying out a procedure on a part of the patient;
the tool support member having a second end including an actuation device for actuating the operating device;
the tool being formed of a material which:
   has a minimal impact on MR images due to the lack of MR spin signal in the material;
   is non-ferromagnetic so as to be unresponsive to a magnetic field of the MR imaging system;
   is non-conductive of electric current so as to be unresponsive to an RF field of the MR imaging system so as to avoid heating of the tool by the RF field;
wherein the tool support member is carried on a tool holder and wherein there is provided a force limiting component arranged to limit bending force applied to the tool support member by the tool holder to a predetermined maximum force;
wherein the force limiting component comprises a joint between two parts of the tool support member which move from an aligned position to a bent position in response to a bending force on the tool support member greater than said predetermined maximum.

According to a fourth aspect of the invention there is provided surgical tool for use on a patient in an MR Imaging system comprising:
a tool support member;
the tool support member having a first end carrying an operating device for carrying out a procedure on a part of the patient;
the tool support member having a second end including an actuation device for actuating the operating device;
the tool being formed of a material which:
   has a minimal impact on MR images due to the lack of MR spin signal in the material;
   is non-ferromagnetic so as to be unresponsive to a magnetic field of the MR imaging system;
   is non-conductive of electric current so as to be unresponsive to an RF field of the MR imaging system so as to avoid heating of the tool by the RF field;
wherein the operating device comprises a biopsy tool which includes cooperating jaws having a fixed jaw and a movable jaw, one of the jaws having a raised contact area fully surrounding a cup for receiving a biopsy sample with the contact area arranged to engage a cooperating surface of the other of the jaws, the jaws being arranged to provide an even application of closing force around the contact area between the jaws.

A control system enables the biopsy tool to acquire a tissue sample in an automated manner.

If the tool is not used in a robotic operating system, a patient stabilizing system can be used that provides rigid support of the patient relative to the biopsy tool.

The biopsy tool does not degrade the performance of the imaging system in relation to image quality by its proximity to the tissue of interest.

The biopsy tool does not degrade image quality of MRI through mechanisms of magnetic susceptibility artefacts. This is accomplished by selecting materials that have similar values of magnetic susceptibility to that of tissue.

The biopsy tool position/orientation can be registered with an imaging system (common coordinate spaces for planning, monitoring, action). Registration maps physical patient-space and the image-space. This can be accomplished through knowledge of the robotic articulated joints and the robotic anchorage position relative to the patient. Real-time targeting may be accomplished by: selectively imaging thin volumes near the tip of the tool; optimizing a segmentation algorithm to isolate the fiducials in the instrument; registering the physical coordinates of the fiducials to their image coordinates; tracing a planned trajectory to the region of interest avoiding specified areas; coordinating instrument movement during the MRI scanner acquisition stage so as to not interfere with image quality.

An instrument and imaging system is provided that maintains an updated image volume set of multi-parametric images (T1, T2, DTI etc) over the course of the procedure. Procedures include: biopsy, tumor resection. Reasons to update the image set include: patient movement, removal of tumor, brain shift.

The tool is invisible in the MR image after the point of contact with the robot end-effector. This is accomplished by material selection (e.g. ceramics) and modification to match magnetic susceptibility of human tissue.

The tool is robust for clinical use despite being made of brittle materials such as ceramics.

The surgeon has full control of biopsy tool when it is in the go/no-go zone.

The biopsy tool force is limited to that necessary to acquire a specimen.

Full movement of the effector shall be greater than the full movement of the tip. This ensures that the tip can be fully closed after positioning. Full stop is possible in one direction. This tool allows the friction actuation to be re-set (this is not a calibration).

The tool is designed to be easy to manufacture, assemble, and refurbish; the tool accommodates fluid flushing for cleaning by a hole in the PEEK tube that connects to the ceramic tube. The tool can be flushed with air or a cleaning solution; The tool is heat sterilizable as per normal hospital sterilization procedures. The tool is designed to be robust for extended use as a re-usable / sterilized surgical instrument.

It is intended for use with a robotic surgery device which provides a movable support with at least 6 degrees of freedom. It has a means for retention of a biopsy specimen, that is a closed-mouth capture.

The cable actuation method is suitable for use in the MRI scanner and is (MR compatible based on the use of a cable material such as Kevlar/Vectran) and a spring material such as titanium, that does not interfere with imaging quality in that it is RF quiet and has a safety mechanism by using a high cable tension which causes slip by design to prevent shattering of the ceramic hollow cylinder of the tool support.

The biopsy sample acquisition method does not rely on sharp edges of the jaw. The even application of closing force on the jaw and the complete mating/sealing of the upper and lower jaw contact areas ensures sample. This feature reduces manufacturing/refurbishing costs and increases tool robustness/reliability.

The tool provides a means for retention of a biopsy specimen as well as for cutting / dissecting tissue. Mechanical activation of the device opens and closes a mouth on the end of the shaft. It is an effective cutter / dissector.

The tool does not introduce imaging artefacts by its presence in the image (e.g. magnetic susceptibility artefacts). This is of particular importance for the biopsy tool tip which is positioned close to the point of sample acquisition

The tool is integrated with the robotic system to localize its position in physical space via known geometry, joint orientations, and relative position offsets from the robot base. Biopsy sample location is stored in system memory as a virtual landmark. Further clinical actions can be taken with precise reference to where the biopsy sample was taken.

The tool is part of a complete system for planning, analysis (pathology type), treatment selection, treatment and assessment.

Used in conjunction with MRI images, the images are spatially registered to the tool and the biopsy tool can be precisely moved to the location of the desired sample. This ensures an industry standard stereotactic accuracy of approximately 1-2mm.

The biopsy tool can be used in two modes of operation at the discretion of the surgeon. One is a manual mode, the other is an automated mode and depends on integration of the surgical robot, tool and MR imaging system.

Mode A: mouth is closed on entering cavity. Surgeon opens mouth, moves it forward and closes it to acquire sample.

Mode B: Auto-biopsy. Robotic automated control of mouth and forward motion once the surgeon positions the tool initially. Benefit is smooth controlled motion.

It therefore has designed slip couplings and bend joints to prevent overloading and a sample collection device which avoids excessive force. It has cleaning ports integrated into the design so that sterility can be obtained by flushing the device interior with a cleaning fluid. A novel spring-loaded capstan ensures proper cable tension. A unique jaw shape enables a cutting pressure to be applied simultaneously around the desired tissue and does not depend on sharp edges to obtain the sample. Springs in the main casing provide cable tensioning to keep the jaws in a default closed position for movement of the biopsy device along a trajectory to the sample to be acquired.

### BRIEF DESCRIPTION OF THE DRAWINGS

One embodiment of the invention will now be described in conjunction with the accompanying drawings in which:
Figure 1 is a schematic side elevational view of a microsurgical robot system operating with the bore of an MR magnet and including a tool according to the present invention.
Figure 2 is a schematic side elevational view of one robot arm of the system of Figure 1 with the tool according to the present invention.
Figure 3 is a side elevational view of the arm of Figure 2 on an enlarged scale with the tool according to the present invention.
Figure 4 is a schematic illustration of an MR image and visual image controlled micro-surgery system.
Figure 5 is an isometric view of the tool of Figure 1.
Figure 6 is an isometric view of the actuation portion only of the tool of Figure 5 shown sectioned along a vertical center line.
Figure 7 is an isometric view of the actuation portion only of the tool of Figure 5 shown sectioned along a horizontal center line.
Figure 8 is a longitudinal cross-sectional view of the operating portion only of the tool of Figure 5.
Figure 9 is a photograph showing the jaws of the operating portion of the tool of Figure 8.

In the drawings like characters of reference indicate corresponding parts in the different figures.

### DETAILED DESCRIPTION

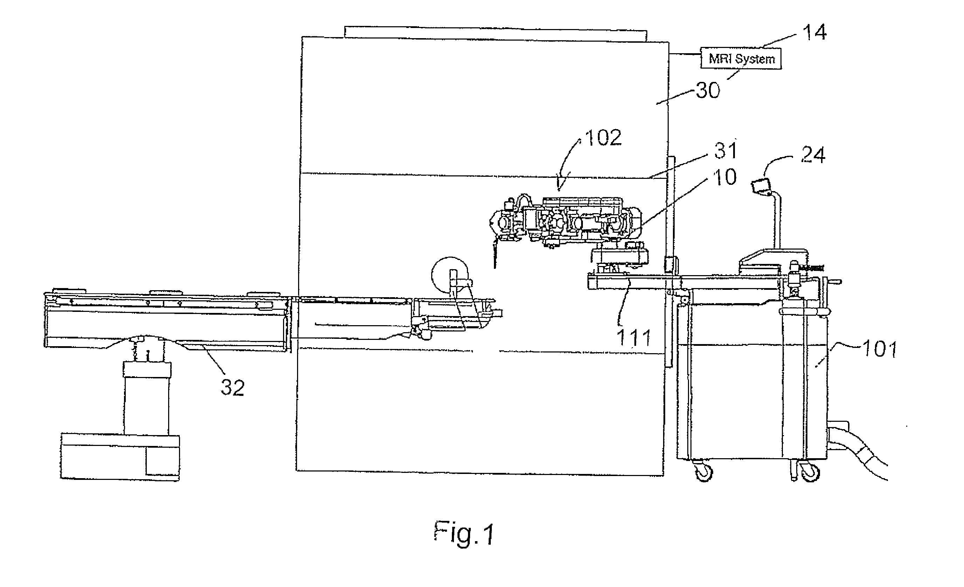

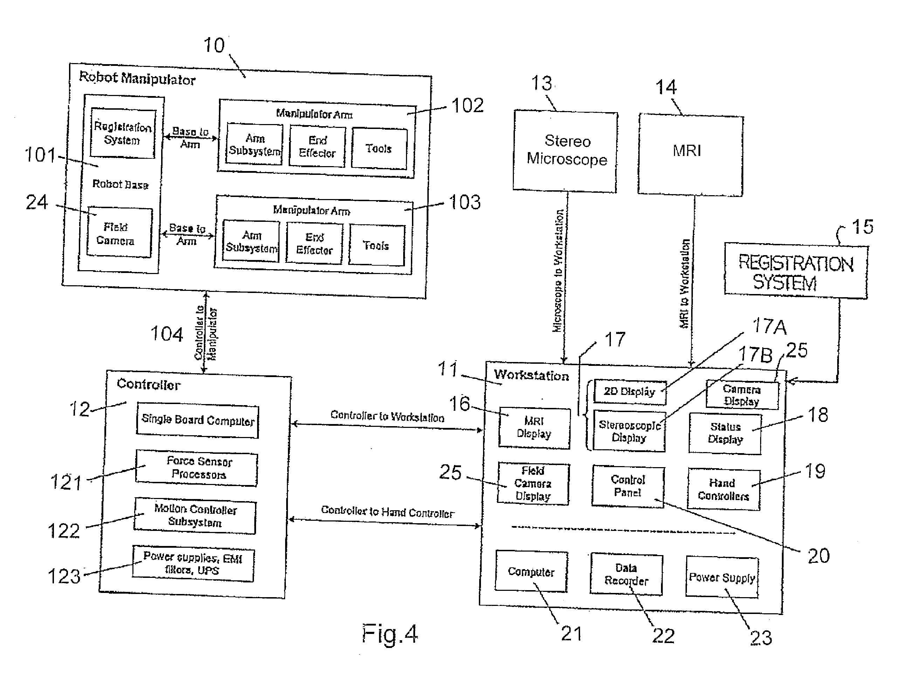

An overview of the system is shown in Figures 1 to 4 which comprises a robot manipulator 10, a work station 11 and a controller 12 which communicates between the robot manipulator and the work station. As an input to the work station is also provided a stereo microscope 13, an MRI imaging system 14 and a registration system 15.

The work station includes a number of displays including at first display 16 for the MRI image, a second display 17 for the microscope image and a third display 18 for the system status. Further the work station includes two hand controllers schematically indicated at 19 and an input interface or control panel 20 allowing the surgeon to control the systems from the work station while reviewing the displays. The work station further includes a computer or processor 21, a data recording system 22 and a power supply 23.

The display 17 includes a stereoscopic display 17A which provides a simulated microscope for viewing the images generated by the stereo-microscope system 13. Further the display 17 includes a monitor 17B which displays a two dimensional screen image from the microscope system 13.

The robot manipulator 10 includes a field camera 24 which provides an image on a monitor 25 at the work station.

The magnetic resonance imaging system 14 is of a conventional construction and systems are available from a number of manufacturers. The systems are of course highly complicated and include their own control systems so that the present workstation requires only the display of the image on the monitor 16 where that image is correlated to the position of the tool using known registration systems.

The hand controllers 19 are also of a commercially available construction available from a number of different sources and comprise 6 degrees of freedom movable arms which can be carefully manipulated by the surgeon including end shafts which can be rotated by the surgeon to simulate the rotation of the tool as described hereinafter. An actuator switch on the tool allows the surgeon to operate the actuation of the tool on the robot as described hereinafter.

The robot manipulator comprises a cabinet 101 and two arms 102 and 103 which are mounted on the cabinet together with the field camera 24 which is also located on the cabinet. The field camera is mounted at the back of the cabinet viewing past the arms of the front of the cabinet toward the patient and the site of operation to give a general overview field of the situation for viewing on the display 25.

The control system 12 for communication between the work station and the robot manipulator and for controlling the operation of each of those components includes a force sensor sub system 121 and a motion control sub system 122 together with power supplies and further components as indicated schematically at 123. The force sensor sub system controls the feed back forces as detected at the end effector of the robot arm to the hand control systems 19. The motion control subsystem 122 converts the motion control sensors from the hand-control system 19 into individual operating instructions to the various components of the arms. The motion control sub system also provides an output which is communicated to the work station for display on the MRI imaging monitor 16 of the location of the tip of the tool relative to the image displayed on the screen 16, as generated by the registration system 15.

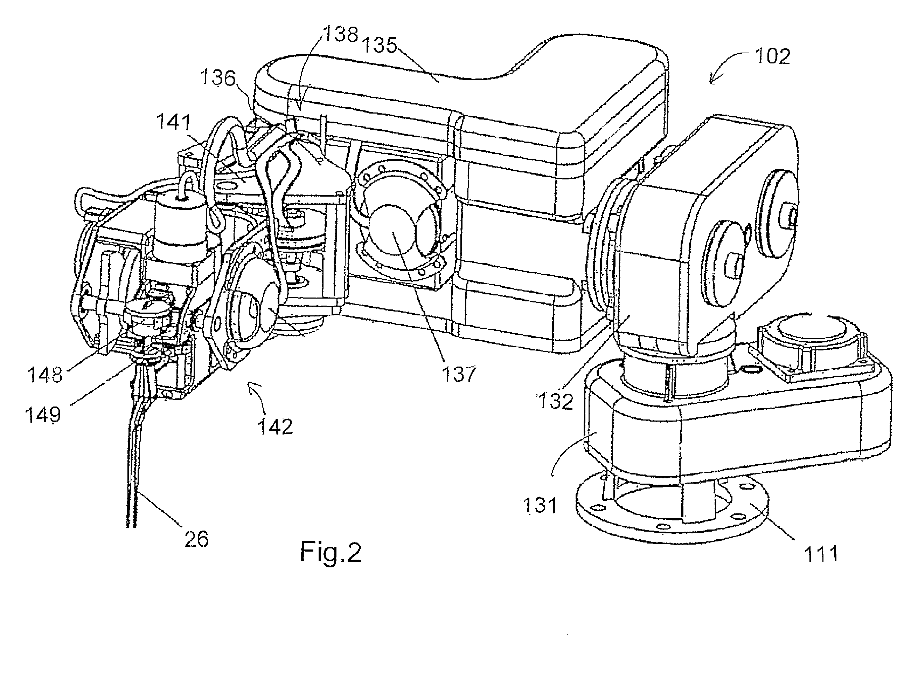

The structure of the arms is shown in Figure 2, where the arms are mounted with their base 111 for attachment to the cabinet support. Each of the arms 102 and 103 includes a number of joints which allow operation of a tool schematically indicated at 26. Thus each arm includes a first joint defining a shoulder yaw pivot 131 defining a vertical axis of rotation. On the vertical axis is mounted a second joint 132 forming a shoulder roll joint which provides rotation around a horizontal axis. The shoulder yaw axis extends through the joint 132. A rigid link 135 extends from the joint 132 to an elbow joint 136 which is cantilevered from the shoulder roll joint 132. The elbow joint includes an elbow yaw joint 137 and an elbow roll joint 138. The yaw joint 137 is connected to the outer end of the link 135 and provides rotation about a vertical axis. The roll joint 138 is located on the axis and provides a horizontal axis. A link 141 lies on the horizontal axis and extends outwardly from the joint 138 to a wrist joint generally indicated at 142. The wrist joint 142 includes a wrist yaw joint and wrist roll joint. The wrist yaw joint provides a vertical axis about which a link can pivot which carries the roll joint. The roll joint provides a horizontal axis which allows the tool 26 to rotate around that horizontal axis. The tool 26 includes a roll joint 148 including a gear drive 150 which provides rotation of the tool 26 around its longitudinal axis by driving a gear of the tool. The tool further includes a tool actuator 149 which is grasped by one jaw 149A of the actuator of the robot and can move longitudinally along the tool relative to the joint 148 which is grasped by a jaw 148A of the robot to provide actuation of the tool using various known tool designs. That is the jaws 148A and 149A of the robot move longitudinally of the tool to effect the operation of the tool.

Thus the forces required to provide rotation around the various axes are minimized and the forces required to maintain the position when stationary against gravity are minimized. This minimization of the forces on the system allows the use of MRI compatible motors to drive rotation of one joint component relative to the other around the respective axes.

The arrangement described above allows the use of piezoelectric motors to drive the joints. Such piezoelectric motors are commercially available and utilize the reciprocation effect generated by a piezoelectric crystal to rotate by a ratchet effect a drive disc which is connected by gear coupling to the components of the joint to effect the necessary relative rotation.

The robot therefore can be used in the two arm arrangement for microsurgery in an unrestricted area outside of the closed bore magnet or for microsurgery within an open bore of a magnet where the arrangement of the magnet can be suitable to provide the field of operation necessary for the two arms to operate. The two arms therefore can be used with separate tools to effect surgical procedures as described above. In some cases a single arm can be used to effect stereotactic procedures including the insertion of a probe or cannula into a required location within the brain of the patient using the real time magnetic resonance images to direct the location and direction of the tool.

In Figure 1, the system is shown schematically in operation within the bore of a magnet 30 of the MRI system 14. The bore 31 is relatively small allowing a commercially available patient table 32 to carry the required portion of the patient into the bore to the required location within the bore. The field camera 24 is used within the bore for observing the operation of the robot 10 and particularly the tool 26.

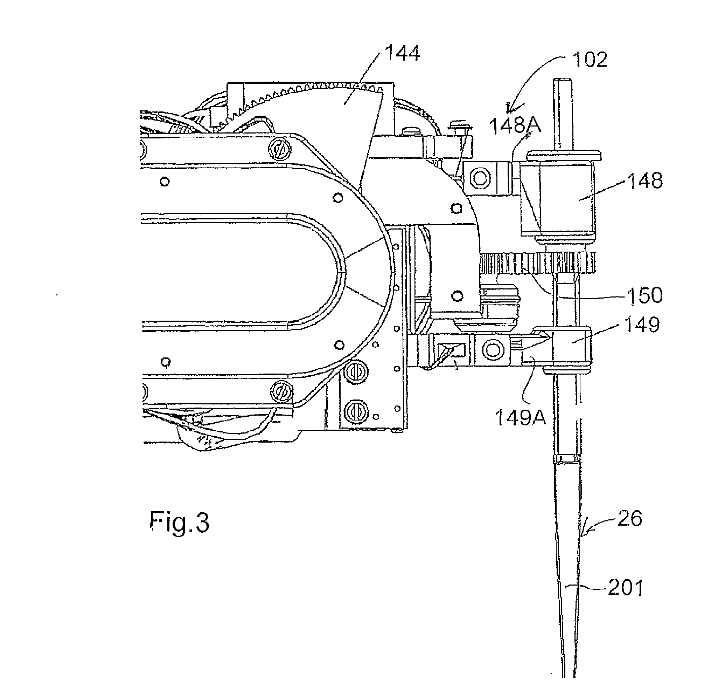

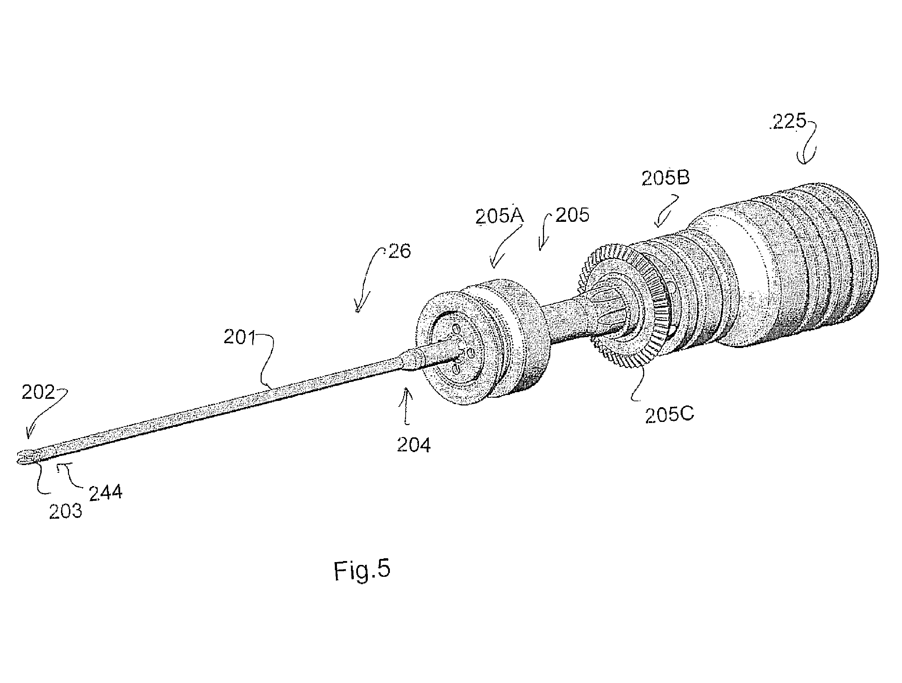

In Figures 5 to 8 is shown the tool 26 of Figure 1 which provides a surgical tool for use on a patient in an MR Imaging system. This comprises a tool support member or shaft 201 having a first end 202 carrying an operating device 203 for carrying out a procedure such as a biopsy on a part of the patient. A second end 204 of the tool support member includes the actuation device 205 including a first actuation portion 205A for engaging the jaw 149A of Figure 3 and a second actuation portion 205B for engaging the jaw 148A of Figure 3 of the robot for actuating the operating device 203. The portion 205B carries the gear 205C for cooperation with the gear 150 of the robot.

In order to be operable during imaging within the bore of the magnet, the tool itself is formed of materials which are non-ferromagnetic so as to be unresponsive to a magnetic field of the MR imaging system, are non-conductive of electric current so as to be unresponsive to an RF field of the MR imaging system so as to avoid heating of the tool by the RF field and have a magnetic susceptibility which is substantially equal to that of human tissue.

The materials selected for manufacture of the part of the tool include a ceramic material. Thus those parts which lie immediately adjacent the human tissue are not formed of titanium since that material has been found to have a magnetic susceptibility which is sufficiently different from that of the human tissue that the MR image includes unacceptable artefacts at the interface with the human tissue, which is of course in many cases the area of most interest.

The ceramic material selected can be Yttrium zirconia, types of alumina, silicon nitride, and alloys thereof.

The tool comprises an axial tube 220 which is carried inside an outer axial tube 221 by bearings 222 and 223 allowing longitudinal sliding movement of the outer tube 221 on the outside surface of the tube 220. The outer tube 221 is connected to the actuation portion 205A so that longitudinal movement of the portion 205A drives the outer tube 221 axially.

The portion 205B and the gear 205C surround the outer tube 221 and are connected to a housing 225 which holds the inner tube 220. Thus the housing 225 is connected to the jaw 148A to hold the tool and the tube 220 at a position determined by the jaw. The tool is rotated by the gear 150 which drives the gear 205C to rotate the housing 225 and thus the tube 220 about the longitudinal axis of the tool. This rotation takes place relative to the portions 205A and 205B which remain stationary on bearings 224 located between the portion 205B and the housing 225 and on bearings 224A located between the portion 205A and the housing outer tube 221. In this way the jaws hold the portions fixed from rotation and allow the gear to rotate the tool including the housing around the axis.

Movement of the jaw 149A longitudinally of the of the tool toward and away from the housing 225 acts to move the outer tube 221 axially which connects to a crank 226 which drives rotation of a rotary capstan member 227 within the housing 225 relative to a transverse shaft or axle 228 at right angles to the longitudinal axis.

The drive system to the operating tool 203 also includes a band or tendon 230 with two longitudinally extending runs 230 and 231 along the inner tube 220.

Thus the tendon or band 230 is driven along its length by the rotary capstan member 227 at the actuation device around which the band is wrapped in two loops each applied into a respective one of two grooves 250 and 251 around the outer surface of the capstan member.

The capstan member is rotated by the crank 226 driven by movement longitudinally of the tool support member. The crank engages one of inner and outer coaxial members 220, 221. The capstan member is housed within the housing 225 connected to the other of the inner and outer coaxial members 220 and 221. Thus the relative longitudinal movement between the tubes 220, 221 driven by the actuators moving the engagement members 205A and 205B drives rotation of the single capstan member around its axis to actuate movement of the tendon 230.

The capstan member is biased along the tool head relative to the housing by a pair of springs 252, 253 each engaged between an inner end face of the housing 225 and the axle 228 of the capstan member 227. Thus the axle 228 is pushed inside the housing along the housing away from the end face of the housing to tension the tendon

The capstan member is driven by an actuation method using the actuators 205A and 205B that does not depend on electricity.

The components described above are formed from different materials of PEEK, titanium. Thus, as well as the ceramic material which is used for the inner axial rod and bearings); the PEEK is used for the outer axial rod 220; titanium is used for the set screws 233 and tension spring); Kevlar/Vectran is used for the tendon 230.

As the tool and particularly the tool support member and the operating device are formed of a material which can crack if subjected to a force greater than said predetermined maximum, it is necessary to provide systems which ensure that the forces applied do not exceed a predetermined maximum..

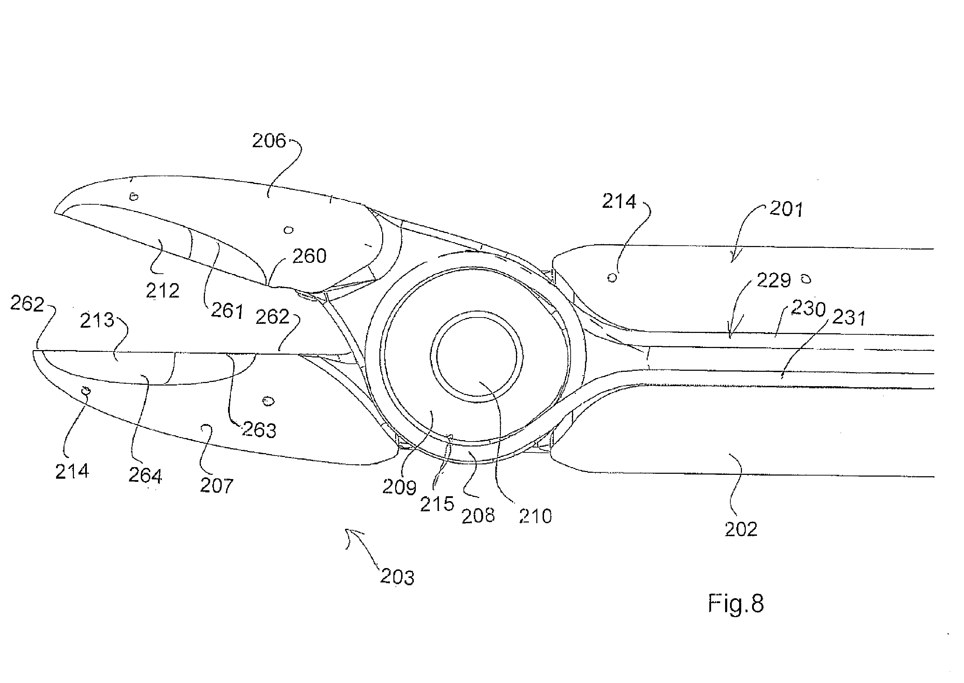

As shown in Figure 8 there is provided a force limiting component arranged to limit force applied to the operating device by the actuation device to a predetermined maximum force. Thus the force limiting component includes a drive transfer member defined by a wrap 208 of the elongate tendon 230 movable along its length which slips on a pulley 209 around which it is wrapped. This allows slippage of drive from the actuation device 149A to jaws 206 and 207 of the operating device 203.

The pulley 209 is connected to the movable jaw 206 to move it in respective direction depending on the direction of movement of the tendon 230 and thus on the pulling action on the tendon 230 effected by the actuator 149. The jaw 206 rotates around a bearing shaft 210 carried on the stationary jaw 207 which is fixed to the shaft 202. Precise control is translated from full travel of the effector 149 into 1/8" of tip movement of the jaws. This is implemented using the co-axial construction of the inner and outer tubes. Pull-back on the effector 149 causes a rotation on the pulley 209. The pulley 209 has a wedge-shape so that motion scaling or reduction occurs. Essentially, linear axial movement of the effector 149 turns the pulley 209. The pulley 209 interacts with the pin 210 as a lever effect. The effective pulley diameter on the actuation tendon 230 is optimal. A 60 degree rotation maps to the effective surface area. The two wedge and half-pulleys arrangement maintain a rigid pulley structure.

Thus the drive transfer member comprises the elongate band or tendon 230 movable along its length by the actuation device provided by the portions 148, 149 of the robot end effector which operate through the portions 205A and 205B. The band is arranged to slip on the drive coupling 209 around which it is wrapped so that as soon as a pre-determined maximum force between the jaws 206 and 207 is reached, the tendon 230 slips and the jaws move no further regardless of additional forces being applied by the end effector of the robot.

The tendon 230 runs are driven along their length by the rotary member 227 which is rotated around the transverse axis by the 226 crank driven by movement of the outer tube 221 longitudinally of the tool support member.

Thus the friction pulley has just enough force to acquire the biopsy sample. This is accomplished by the torque limiting tendon 230 arrangement which will slip by design after a threshold is exceeded.

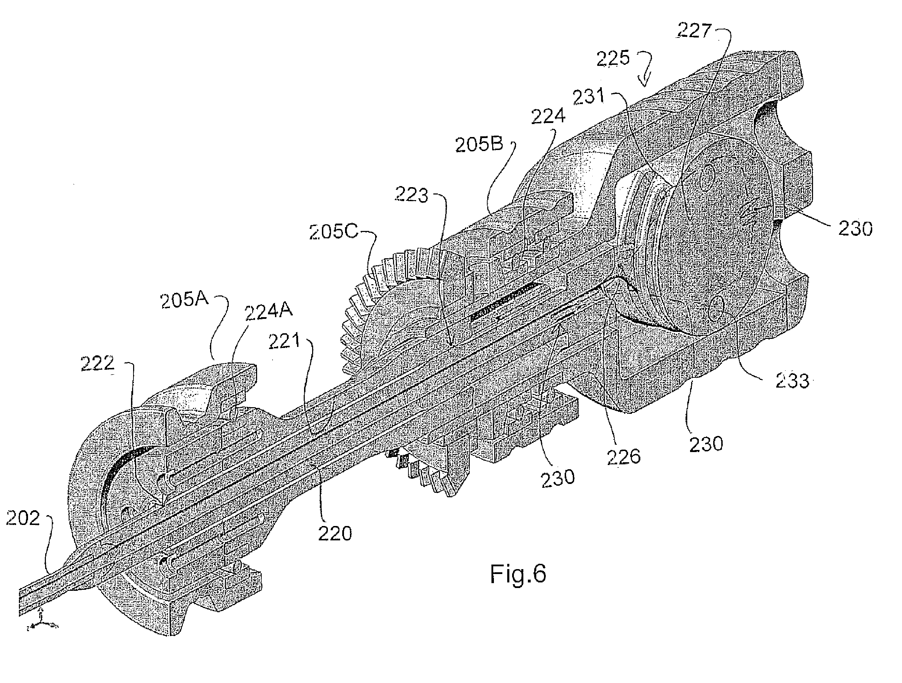

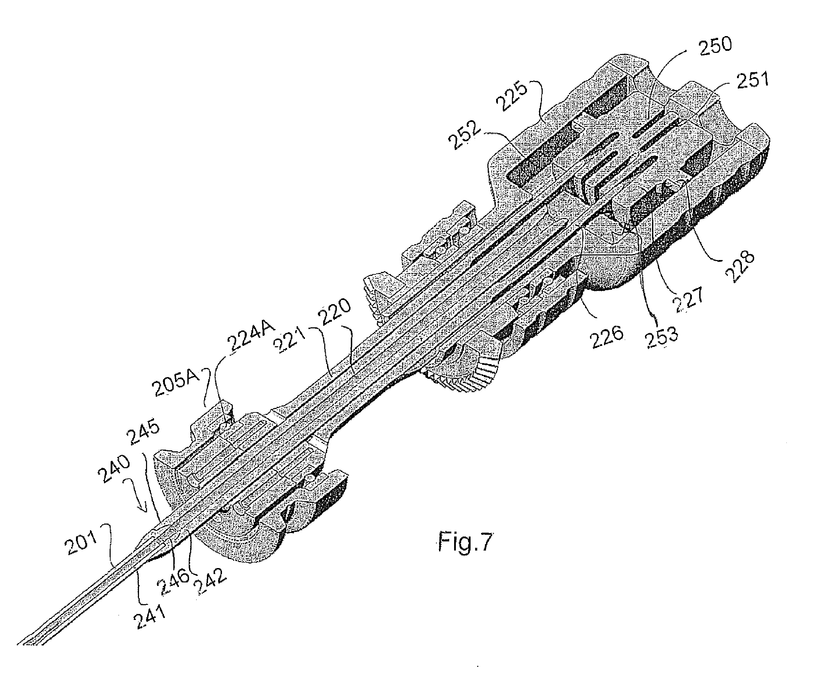

As shown in Figures 6 and 7, the tool support member or shaft 201 includes a force limiting component 240 located between the end 241 of the shaft 202 and the end 242 of the inner tube 220. The joint or component 240 is arranged to limit bending force applied to the tool support member by the tool holder to a predetermined maximum force. That is, if the force applied by movement of the robot arm to the shaft 202 exceeds a predetermined level beyond which cracking or shattering of the shaft 202 and jaw 206 can occur if forced against a stationary object, the joint 240 in the tool support member 202 moves to a bent position in response to a bending force on the tool support member greater than said predetermined maximum.

A second break-away joint 244 is also located at the tip of the shaft 201 and can protect the tool 203 from damage by a similar break away action.

Each break-away joint 240, 244 includes two components 245 and 246 where one provides a convex surface sitting inside a concave surface defined by the other. These surfaces will allow rotation one on the other when the torque between them exceeds the required value. The surfaces are held against one another in frictional contact by the tension in the tendon 230. Thus a deflection of the shaft will elongate the tendon 230. The cable is on the pre-tensioned spring 232. As such, a lateral stiffness of the shaft 202 at the joints 240 and 244 can be defined and configured. The joint 244 at the tip and the joint 240 at the ceramic/body junction include a specific geometry defined by the portions 245 and 246 which is used to support 90 degree snap-back. This geometry provides a joint between ceramic tube and rest of body in which initially this joint has a high load; but after 10 degrees of deflection, the spring compression rate is significantly reduced. The benefit is that a shorter spring 232 may be used since travel is reduced. This leads to a more compact design.

Thus the two joints 240 and 244 in the tool support member are pulled into engagement of the parts in the straight position by the tendon 230 extending along the tool support member, which operates the operating device, where the tendon 230 stretches in length either along its length by a controlled elasticity or at the spring 232 to allow longitudinal movement of the parts into the bent position.

The operating device is driven by the actuation device through an actuation method that does not depend on electricity that could pose a safety hazard to the patient or that might introduce RF noise. The wrap 208 loops around the jaw pulley 209 twice (or more). Both ends of the cable 229 are attached to the actuation pulley 227 of the actuator at the far end as shown in Figure 6 and are preloaded to accomplish the following:
1. The pulley section 209 of the moveable jaw is held laterally inside fixed jaw. tension of tendon 230 provides a seating force to hold the pin in place (as a result of smaller diameter of pin interfacing with the pulley bore;
2. The tendon 230 tension provides enough friction on the jaw to achieve adequate closing force;
3. The friction actuation provides enough slip so the tendon 230 is never over-tensioned.

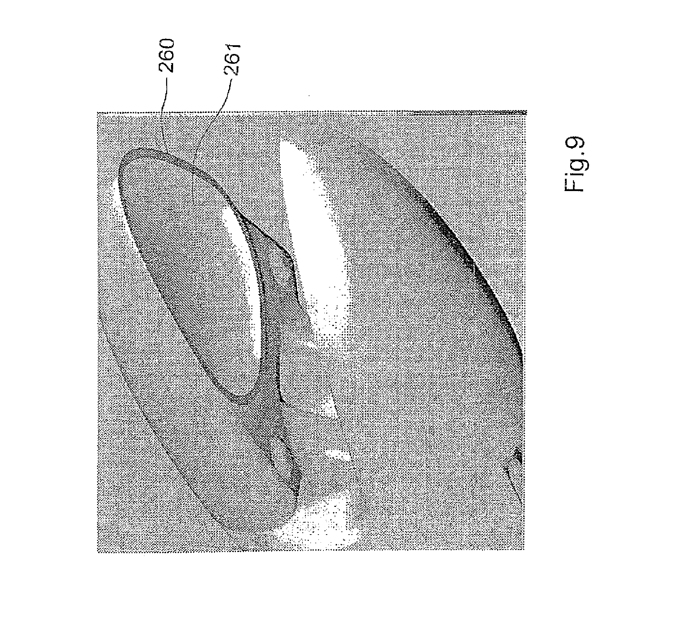

The operating device comprises a biopsy tool which includes the cooperating jaws 206 and 207 having a fixed jaw 207 attached to the member 201 and a movable jaw 206. One of the jaws 206, as best shown in Figure 9, has a raised contact area 260 fully surrounding a cup 261 for receiving a biopsy sample. The contact area defined by the raised lip 260 is arranged to engage a cooperating surface 262 of the other of the jaws which lies in a flat face plane 263 of the jaw 207. The jaws 206 and 207 and the pivot pin are arranged to provide an even application of closing force around the contact area 260 onto the plane 263 between the jaws around the cup 261.

Thus the raised contact area 261 which is circular or oval and defines the edge of the cup 261 when it is closed engages the planar cooperating surface 263 of the other of the jaws 207 so that the raised contact area forms a cutting surface on the planar surface 263 which cuts by pinching around the full periphery of the up rather than by a shearing action. The cutting action thus avoids sharp cutting edges. The planar surface 263 also includes a cup 264 facing and matching the cup 261 of the jaw 206.

The biopsy tool includes a biopsy sample acquisition method that does not rely on sharp edges of a fixed jaw but instead the jaws 206, 207 have a movable jaw 206 which has even application of closing force on the jaw 207 and the complete mating/sealing of the jaw contact area 212, 213. The jaw is closed by the friction pulley 209. The pulley is designed to have a maximum diameter for greatest torque using minimal cable tension. This ensures that the jaw has sufficient closing force to acquire a tissue sample. Minimum cable tension allows the use of a thin cable. The tool implements a torque limit as a safety feature such that the cable will slip by design after a threshold force is exceeded. In this way the friction pulley avoids over-extensions and over-stressing tip and other components. A design feature is that a nominal 90 degree rotation of the pulley maps to a nominal 45 degrees jaw opening angle.

The tip jaw pivot has a large diameter at both ends end but is smaller diameter in the centre where the jaw rotates. Pulley tension keeps the pivot pin correctly positioned.

The pivot construction enables vertical movement of the jaw during closure. This results in a rolling contact of the jaw clipping area due to the back of the jaw closing first, and then the angle of the cable acting on the jaw pulley in such a way as to draw the jaw upward at full closing force. This rolling contact of the jaw ensures that full contact is made around the perimeter of the bite so that full clipping contact is ensured. This full contact enables sample acquisition without relying on sharp edges of the jaw. The ability to acquire a sample from a fairly blunt edge reduces manufacturing cost and is a factor in reliability / durability.

The components are arranged for ease of assembly and disassembly in that the pivot pin 209 has a large diameter at its end 215 but is small in the centre 210 where the jaw rotates. This is not a press fit but rather relies on pulley tension. The benefit is that pivot is kept centred. There is a rolling contact of the two jaws and total contact is made around the perimeter of the bite 212, 213 so that full clipping contact is ensured. This full contact enables sample acquisition but does not rely on sharp edges of the jaw. The ability to acquire a sample from a fairly blunt edge reduces manufacturing cost and is a factor in reliability / durability.

The tension system 232 for the actuation cable 229 is easy to assemble. The cable 229 pulls through a bore defined by a cross bore to the set screw 233. In order to assemble, the process involves compressing the spring 232; turning until the cable engages and cutting cable so that this results in clean and secure cable attachment. In regard to the housing this has a cap which splits in two which allows easier access for install and servicing.

The biopsy tool does not degrade image quality of MRI through mechanisms of magnetic susceptibility artefacts. This is accomplished by selecting materials that have similar values of magnetic susceptibility to that of tissue.

The biopsy tool can be used in two modes of operation at the discretion of the surgeon. One is manual, the other is automated and depends on integration of the surgical robot, tool and MR imaging system.

Mode A: mouth is closed on entering cavity. Surgeon opens mouth, moves it forward and closes it to acquire sample.

Mode B: Auto-biopsy. Robotic automated control of mouth and forward motion once the surgeon positions the tool initially. The benefit is smooth controlled motion.

## Claims

1. A surgical tool for use on a patient in an MR Imaging system comprising:
a tool support member;
the tool support member having a first end carrying an operating device for carrying out a procedure on a part of the patient;
the tool support member having a second end including an actuation device for actuating the operating device;
the tool being formed of a material which:
has a minimal impact on MR images due to the lack of MR spin signal in the material;
is non-ferromagnetic so as to be unresponsive to a magnetic field of the MR imaging system;
is non-conductive of electric current so as to be unresponsive to an RF field of the MR imaging system so as to avoid heating of the tool by the RF field;
has a magnetic susceptibility which is substantially equal to that of human tissue.

2. The surgical tool according to claim 1 wherein there is provided a force limiting component arranged to limit force applied to the operating device by the actuation device to a predetermined maximum force wherein the force limiting component includes a drive transfer member which allows slippage of drive from the actuation device to the operating device.

3. The surgical tool according to claim 2 wherein the drive transfer member comprises an elongate band movable along its length by the actuation device, wherein the band is arranged to slip on a drive coupling around which it is wrapped.

4. The surgical tool according to claim 3 wherein the elongate member is driven along its length by rotary capstan member at the actuation device around which the band is wrapped where the capstan member is rotated by a crank driven by movement of an engagement device longitudinally of the tool support member.

5. The surgical tool according to claim 4 wherein the crank engages one of inner and outer coaxial members with the capstan member housed within a housing connected to the other of the inner and outer coaxial members.

6. The surgical tool according to claim 5 wherein the capstan member is biased along the tool head relative to the housing by a pair of springs engaged between the housing and an axle of the capstan member.

7. The surgical tool according to any one of claims 1 to 6 wherein the tool support member is carried on a tool holder and wherein there is provided a force limiting component arranged to limit bending force applied to the tool support member by the tool holder to a predetermined maximum force.

8. The surgical tool according to claim 7 wherein the force limiting component comprises a joint between two parts of the tool support member which move from an aligned position to a bent position in response to a bending force on the tool support member greater than said predetermined maximum.

9. The surgical tool according to claim 8 wherein the joint in the tool support member is pulled into engagement of the parts in the straight position by a band extending along the tool support member which extends against a spring tension to allow longitudinal movement of the parts into the bent position.

10. The surgical tool according to any one of claims 1 to 9 wherein the tool support member and the operating device are formed at least in part of a ceramic material.

11. The surgical tool according to claim 10 wherein the ceramic material comprises a material selected from the group consisting of Yttrium zirconia, types of alumina, silicon nitride, and alloys thereof.

12. The surgical tool according to claim 10 or 11 wherein the tool is formed of a ceramic material and PEEK with titanium couplings.

13. The surgical tool according to any one of claims 1 to 12 wherein the operating device comprises a biopsy tool which includes cooperating jaws having a fixed jaw and a movable jaw, one of the jaws having a raised contact area fully surrounding a cup for receiving a biopsy sample with the contact area arranged to engage a cooperating surface of the other of the jaws, the jaws being arranged to provide an even application of closing force around the contact area between the jaws.

14. The surgical tool according to claim 13 wherein said raised contact area arranged to engage a planar cooperating surface of the other of the jaws so that the raised contact area forms a cutting edge on the planar surface.

15. The surgical tool according to claim 13 or 14 wherein the planar surface includes a cup facing the cup of said one jaw.
